(19) Europäisches Patentamt
European Patent Office
Office européen des brevets

(11) **EP 2 640 265 B1**

# (12) FASCICULE DE BREVET EUROPEEN

(45) Date de publication et mention de la délivrance du brevet:
**11.08.2021 Bulletin 2021/32**

(21) Numéro de dépôt: **11794555.0**

(22) Date de dépôt: **16.11.2011**

(51) Int Cl.:
*A61B 5/103* *(2006.01)*        *A61B 17/16* *(2006.01)*
*A61B 5/053* *(2021.01)*

(86) Numéro de dépôt international:
**PCT/FR2011/052651**

(87) Numéro de publication internationale:
**WO 2012/066231 (24.05.2012 Gazette 2012/21)**

(54) **SYSTÈME DE DÉTERMINATION DE LA QUALITÉ D'UNE STRUCTURE OSSEUSE D'UN SUJET**

SYSTEM ZUR BESTIMMUNG DER QUALITÄT DER KNOCHENSTRUKTUR EINES INDIVIDUUMS

SYSTEM FOR DETERMINING THE QUALITY OF AN INDIVIDUAL'S BONE STRUCTURE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.11.2010 FR 1059422**

(43) Date de publication de la demande:
**25.09.2013 Bulletin 2013/39**

(73) Titulaire: **SPINEGUARD**
**94160 Saint Mandé (FR)**

(72) Inventeurs:
• **BOURLION, Maurice**
**F-42400 Saint-chamond (FR)**
• **BETTE, Stéphane**
**Tiburon 94920 (US)**

(74) Mandataire: **Plasseraud IP**
**66, rue de la Chaussée d'Antin**
**75440 Paris Cedex 09 (FR)**

(56) Documents cités:
WO-A1-03/068076          WO-A1-2005/102183
WO-A1-2009/152244        WO-A2-2008/119992
DE-A1-102004 035 001     US-B1- 6 997 883

• **HEIKO KOLLER ET AL: "In vitro study of accuracy of cervical pedicle screw insertion using an electronic conductivity device (ATPS part III)", EUROPEAN SPINE JOURNAL, SPRINGER, BERLIN, DE, vol. 18, no. 9, 3 juillet 2009 (2009-07-03), pages 1300-1313, XP019761660, ISSN: 1432-0932, DOI: DOI:10.1007/S00586-009-1054-1**
• **None**

## Description

L'invention se rapporte à un système de détermination de la qualité d'une structure osseuse d'un sujet, notamment de la porosité d'une structure osseuse.

Dans le domaine médical, lors d'une intervention chirurgicale menée sur un patient comme sujet, le chirurgien intervient souvent dans des structures anatomiques complexes, composées de plusieurs éléments, par exemple dans une structure osseuse composée d'une matrice osseuse qui contient des éléments minéraux, et de moelle osseuse, lieu de formation des cellules sanguines, ou dans un disque intervertébral composé d'une matrice de cartilage fibreux ou gélatineux et d'eau.

Il est utile au chirurgien de pouvoir apprécier de façon objective la qualité de ces structures.

En particulier, lors d'une intervention sur une structure osseuse consistant à mettre en place un implant, par exemple une vis dans une vertèbre ou un clou dans un fémur, la tenue de cet implant dépend grandement de la qualité de l'ancrage et donc de la qualité de la structure osseuse dans laquelle il est inséré. En effet, l'implant installé dans une structure osseuse de mauvaise qualité, notamment poreuse, aura une tenue moindre que le même implant installé dans une structure osseuse de bonne qualité.

Par ailleurs, après l'intervention le bon ancrage de l'implant conditionnera une meilleure consolidation, une meilleure fusion, et surtout une diminution des risques associés à un mauvais ancrage de l'implant dans la structure osseuse, comme par exemple l'apparition d'une pseudarthrose génératrice de douleurs ou la nécessité d'une nouvelle intervention en cas de migration de l'implant.

D'autre part, avec le vieillissement de la population, la tendance est de traiter des patients de plus en plus âgés donc ayant des os moins solides. De nouveaux implants, tels que des vis à expansion, dédiés à des os fragiles ne présentant pas une structure osseuse de bonne qualité apparaissent sur le marché. Si le chirurgien connaît, de façon objective, la qualité de la structure osseuse dans laquelle il intervient, il peut adapter sa méthode thérapeutique en conséquence. Par exemple pour l'implantation d'une vis dans une vertèbre, il peut décider, si la structure osseuse n'est pas de bonne qualité, soit de choisir un diamètre de vis plus important, soit d'utiliser un implant spécifique, soit d'injecter un ciment de consolidation, soit de traiter une vertèbre supplémentaire, soit de mettre en oeuvre toute autre méthode qu'il jugerait utile.

On connaît des techniques utilisées préalablement à l'intervention chirurgicale pour donner une indication au chirurgien de la qualité de la structure osseuse.

Par exemple, la méthode DEXA (Dual Energy X-ray Absorptiometry) permet de mesurer la densité osseuse de différentes régions du squelette. Il s'agit de mesurer l'atténuation de deux faisceaux de rayon-X d'énergies différentes à travers les tissus (mous et durs : organes et os). Une fois l'atténuation connue, la densité des tissus traversés est déduite en utilisant une équation de l'atténuation, selon la loi de Beer-Lambert. Les zones examinées peuvent être le corps entier ou des parties de celui-ci, et notamment la colonne vertébrale, la hanche, le col du fémur et l'avant-bras (radius). Les résultats de cet examen sont notés sous la forme d'un score décrivant la densité minérale osseuse mesurée en comparaison avec la valeur normale correspondante de la zone en question. Cet examen permet d'évaluer ainsi le risque de fractures même avant l'apparition d'une première fracture. Il est aujourd'hui considéré comme standard dans la recherche d'ostéoporose chez la femme âgée.

On connaît, par ailleurs, de la demande WO 2008/119992, une technique consistant, dans un premier temps, à appliquer et mesurer sur une partie du corps des signaux électriques alternatifs émis sur une plage de fréquence étendue puis, dans un deuxième temps, à traiter les signaux électriques pour déterminer l'impédance et le décalage de phase pour chacune des fréquences et en déduire une valeur de la densité osseuse de la partie du corps.

Ces techniques non-invasives et indolores qui procèdent d'un examen rapide offrant de bonnes performances en termes de précision et de reproductibilité présentent, toutefois, l'inconvénient principal de proposer un examen global étudiant à la fois l'os cortical, de densité importante, et l'os spongieux de faible densité.

Or comme il peut y avoir une grande hétérogénéité, en termes de qualité, dans une structure osseuse (par exemple une vertèbre peut être de moindre qualité qu'une autre vertèbre voisine pour un même patient) il ne suffit pas de connaître la qualité globale de la structure osseuse. Une information locale est nécessaire.

Pour obtenir une information locale sur la qualité de la structure osseuse, on connaît la biopsie osseuse qui consiste à prélever, généralement sous anesthésie, un petit fragment d'os pour analyser sa structure. La zone de ponction est en principe l'os de la hanche (l'os iliaque).

Toutefois, cette technique présente un certain nombre d'inconvénients parmi lesquels la nécessité de faire subir une intervention douloureuse supplémentaire au patient et l'impossibilité d'extrapoler à l'ensemble de la zone sur laquelle le chirurgien doit intervenir. De plus, l'obtention d'information sur la qualité de la structure osseuse nécessite un traitement du fragment prélevé et n'est donc pas immédiat.

Outre les inconvénients évoqués précédemment, les techniques précitées sont préopératoires et augmentent le nombre et la durée des interventions subies par le patient.

Or lors d'une intervention chirurgicale, il est critique que le temps opératoire soit le plus court possible pour diminuer les risques (anesthésie, infections...). En conséquence, la mesure de qualité locale de la structure osseuse se doit d'être obtenue sans augmentation du temps opératoire.

On connaît également du document US 6 997 883 un système dentaire de diagnostic. Le système comprend un corps adapté pour être inséré dans un trou préformé dans une dent et un dispositif de mesure de résistance comprenant deux électrodes pour distinguer un tissu sain de la dent d'un tissu abîmé de la dent à partir d'une différence de conductivité résultant d'une différence de remplissage de la dent par un liquide. Ce système qui s'applique à une dent et non à une structure osseuse nécessite un forage préalable de la dent et un remplissage préalable de la dent par un liquide. De plus, la détermination de la qualité du tissu est réalisée de manière relative à partir d'une variation de conductivité.

Le document WO 2009/152244 décrit un système de détermination des dimensions d'un trou dans une structure osseuse comprenant également un corps adapté pour être inséré dans un trou préformé.

Par ailleurs, dans les deux derniers systèmes précités, les électrodes sont en contact avec un tissu qui n'est pas l'os intact dont on souhaite mesurer la qualité mais plutôt avec un matériau résultant du mélange de débris osseux créés avec le précédent perçage et de fluides présents, par exemple du sang. Or le mélange résultant n'a pas la même qualité que l'os intact avant le perçage. Par conséquent, ces systèmes ne permettent pas de déterminer en continu, notamment lors du forage de la structure osseuse, la qualité d'une structure osseuse.

Le document DE 10 2004 035 001 décrit, par ailleurs, un système de découpe d'une structure osseuse dans lequel un dispositif de traitement est adapté pour fournir à un utilisateur une estimation d'une épaisseur restante à partir d'une variation d'impédance représentative d'un changement de tissu.

Or il existe un besoin pour un système de détermination de la qualité de la structure osseuse permettant au chirurgien de pouvoir apprécier de façon objective la qualité de cette structure, localement, directement au contact de l'os, en temps réel et de façon continue lors du forage de la structure osseuse.

L'invention propose un système de détermination de la qualité d'une structure osseuse d'un sujet, notamment de la porosité d'une structure osseuse, ledit système comprenant :

- un corps adapté pour réaliser un forage de la structure osseuse et présentant une surface extérieure,
- au moins une première électrode présentant une surface de contact agencée sur la surface extérieure du corps pour venir en contact avec la structure osseuse lors du forage,
- au moins une deuxième électrode présentant une surface de contact agencée sur la surface extérieure du corps pour venir en contact avec la structure osseuse à distance de la première électrode lors du forage,
- un générateur électrique adapté pour appliquer pendant une durée déterminée un courant électrique entre les surfaces de contact des première et deuxième électrodes, ledit courant électrique présentant une caractéristique choisie parmi une tension et une intensité qui est connue,

- un dispositif de mesure adapté pour mesurer en continu pendant la durée déterminée l'autre caractéristique choisie parmi la tension et l'intensité du courant électrique traversant la structure osseuse entre les surfaces de contact des première et deuxième électrodes,
- un dispositif de traitement adapté pour déterminer en continu pendant la durée déterminée une grandeur électrique représentative de l'aptitude de la structure osseuse à laisser passer le courant électrique entre les surfaces de contact des première et deuxième électrodes à partir de la caractéristique connue et de la caractéristique mesurée, et pour délivrer en continu pendant la durée déterminée un signal représentatif de la qualité de la structure osseuse, notamment de la porosité de la structure osseuse, entre les surfaces de contact des première et deuxième électrodes à partir de la grandeur électrique déterminée, le dispositif de traitement étant adapté pour calculer en continu pendant la durée déterminée une valeur pour la grandeur électrique et pour associer en continu pendant la durée déterminée la valeur calculée pour la grandeur électrique à une valeur pour la qualité de la structure osseuse, notamment pour la porosité de la structure osseuse.

Ainsi, selon l'invention, les première et deuxième électrodes placées sur le corps adapté pour le forage de la structure osseuse viennent directement en contact avec la portion de la structure osseuse dont la qualité doit être déterminée. L'invention permet de mesurer localement, en temps réel et en continu, c'est-à-dire pendant toute la durée déterminée correspondant par exemple à la durée du forage ou de l'intervention chirurgicale, une grandeur représentative de l'aptitude à laisser passer le courant électrique pour pouvoir en déduire en temps réel la qualité de cette structure. Cette détermination de la qualité de la structure osseuse peut être mise en œuvre au cours de l'intervention chirurgicale, notamment lors du forage de la structure osseuse, mais également, dans une application particulière, après l'intervention, pendant la période de guérison, en vue d'assurer un suivi de la reconstruction osseuse.

En effet, l'aptitude d'un matériau donné à laisser passer le courant électrique, représentée notamment par la conductivité ou la résistivité du matériau, est une propriété intrinsèque du matériau qui dépend de la nature de ce matériau.

D'un point de vue macroscopique, la structure osseuse est un matériau composite principalement composé de deux phases : l'os solide peu conducteur et la moelle osseuse contenant des cellules du sang conductrice. Chacune de ces deux phases ont des aptitudes à laisser passer le courant électrique distinctes. La mesure de l'aptitude globale de la structure osseuse à laisser passer le courant électrique permet d'obtenir une information sur la proportion de ces deux phases et donc sur la qualité de la structure osseuse. Dans une structure osseuse très dure comme l'os cortical, l'aptitude à laisser passer le courant électrique sera faible parce que la phase solide, peu conductrice, y est prépondérante alors que l'aptitude à laisser passer le courant électrique sera plus élevée dans un os ostéoporotique étant donné une plus forte présence de moelle osseuse, très conductrice.

Ainsi, plus la structure osseuse présentera une aptitude élevée à laisser passer le courant électrique, plus la structure osseuse sera poreuse et sera considérée comme présentant une mauvaise qualité.

Selon des dispositions particulières, le système selon l'invention trouve des applications avantageuses dans le domaine de la chirurgie de fusion (reconstruction) osseuse, notamment pour le suivi et la confirmation de la fusion osseuse. L'invention peut en effet permettre de mesurer de l'état de la fusion osseuse pour en suivre l'évolution.

Un tel suivi présente un grand intérêt dans la mesure où une mauvaise fusion osseuse peut provoquer des douleurs dues à une mobilité non souhaitée pouvant induire une nouvelle chirurgie. Par ailleurs, afin de maintenir une contrainte sur l'os au cours de la fusion, il est utile de pouvoir adapter la mobilité, notamment en jouant sur la rigidité de l'implant (par exemple tige ou plaques de fusion, fixateurs externes que l'on ajuste au cours de la fusion) ou sur le réglage ou le retrait d'une orthèse (par exemple corset de soutien) ou l'ajustement d'un programme de rééducation fonctionnelle. Il peut être utile, quand une stimulation de croissance osseuse est mise en place, de pouvoir adapter cette stimulation à l'évolution de la fusion.

Ainsi, avec l'invention, lors de la phase de guérison pendant laquelle les structures osseuses vont fusionner, le chirurgien peut s'assurer de l'évolution de la fusion, afin d'adapter son traitement (implant à rigidité variable, ou stimulation de croissance osseuse, adaptation et retrait du corset).

Dans un mode de réalisation, le générateur électrique peut être adapté pour appliquer le courant électrique sous la forme d'au moins une impulsion électrique.

Par ailleurs, le dispositif de traitement peut être adapté pour mémoriser une fonction de transfert reliant chacune des valeurs d'une plage de valeurs pour la grandeur électrique à une valeur d'une plage de valeurs pour la qualité de la structure osseuse, notamment pour la porosité de la structure osseuse.

Le dispositif de traitement peut être adapté pour établir un premier rapport entre la caractéristique connue et la caractéristique mesurée, et pour déterminer la grandeur électrique à partir dudit premier rapport.

On peut notamment prévoir que la grandeur électrique soit choisie parmi la conductivité et la résistivité de la structure osseuse entre les surfaces de contact des première et deuxième électrodes, le dispositif de traitement étant adapté pour :

- déterminer une distance entre les surfaces de contact des première et deuxième électrodes,
- déterminer des dimensions des surfaces de contact des première et deuxième électrodes avec la structure osseuse,
- établir un deuxième rapport entre ladite distance et lesdites dimensions, et
- calculer la grandeur électrique à partir des premier et deuxième rapports.

Une aptitude faible à laisser passer le courant électrique pourra alors être caractérisée par une conductivité faible ou, inversement, une résistivité élevée. Et une aptitude élevée à laisser passer le courant électrique pourra être caractérisée par une conductivité élevée ou, inversement, une résistivité faible.

Le dispositif de traitement peut être adapté pour mémoriser la distance entre les surfaces de contact des première et deuxième électrodes, et les dimensions des surfaces de contact des première et deuxième électrodes avec la structure osseuse.

La surface de contact d'au moins l'une des première et deuxième électrodes peut présenter une dimension supérieure à 400 μm.

Le dispositif de traitement peut être adapté pour mémoriser un ensemble de valeurs pour la grandeur électrique sur la durée déterminée. Une telle disposition permet d'enregistrer l'évolution de la qualité osseuse au cours d'une mesure pour en permettre un traitement ultérieur.

En outre, le corps peut s'étendre selon un axe central jusqu'à une extrémité distale, au moins l'une des première et deuxième électrodes s'étendant parallèlement à l'axe central, la surface de contact de ladite électrode affleurant la surface extérieure du corps à l'extrémité distale.

Au moins l'une des première et deuxième électrodes peut s'étendre à l'intérieur du corps jusqu'à une extrémité libre présentant la surface de contact, ledit corps comportant une couche de matière isolante entourant ladite électrode de telle sorte que seule ladite surface de contact affleure la surface extérieure du corps.

Le système tel que défini précédemment peut comprendre un boîtier solidarisé au corps et un dispositif d'alimentation alimentant en énergie électrique au moins le générateur électrique et le dispositif de mesure, ledit boîtier étant adapté pour recevoir au moins le générateur électrique, le dispositif de mesure et le dispositif d'alimentation.

Dans un mode de réalisation, le boîtier forme une poignée depuis laquelle s'étend le corps.

On peut prévoir que le boîtier et le corps soient solidarisés de manière amovible, le corps étant réalisé en un matériau biocompatible, implantable dans la structure osseuse, la surface extérieure du corps étant adaptée pour permettre l'ancrage du corps dans la structure osseuse, le boîtier comportant des organes de connexion électrique du générateur électrique et du dispositif de mesure aux première et deuxième électrodes.

Le corps peut ainsi former un implant permettant notamment d'améliorer le suivi de la fusion osseuse. Le boîtier, par exemple externe c'est-à-dire placé à l'extérieur du sujet, peut assurer l'application et la mesure du courant électrique en étant connecté électriquement aux première et deuxième électrodes, implantées.

Dans un autre mode de réalisation, le boîtier et le corps sont réalisés en un matériau biocompatible, implantable à l'intérieur du sujet.

Dans ce mode de réalisation, ce sont à la fois le boîtier enfermant les composants nécessaires à l'application et à la mesure du courant électrique, et le corps qui forment un implant notamment en vue d'améliorer le suivi de la fusion osseuse.

De façon avantageuse, le système peut comprendre une boucle de rétroaction selon laquelle les différents composants du système sont pilotés en fonction du signal représentatif de la qualité de la structure osseuse. Cette disposition peut être particulièrement utile dans le cadre d'un système dont le corps ou le boîtier et le corps sont implantés pour pouvoir ajuster les contraintes mécaniques et/ou électriques lorsqu'une stimulation électrique est prévue sur la structure osseuse.

D'autres objets et avantages de l'invention apparaîtront à la lecture de la description qui suit de modes de réalisation particuliers de l'invention donnés à titre d'exemple non limitatif, la description étant faite en référence aux dessins annexés dans lesquels :

- la figure 1 est une représentation d'un système de détermination de la qualité d'une structure osseuse selon un premier mode de réalisation de l'invention,
- la figure 2 est une représentation d'un instrument de mesure mis en oeuvre dans le système de la figure 1, l'instrument de mesure comprenant notamment une poignée et un corps,
- la figure 3 est une représentation schématique du système de la figure 1, illustrant la connexion d'une première et d'une deuxième électrode dans une partie distale, référencée III sur la figure 2, du corps de l'instrument de mesure, à un générateur électrique et un dispositif de mesure de courant électrique, un dispositif de traitement délivrant un signal représentatif de la qualité de la structure osseuse à partir d'une grandeur électrique,

- la figure 4 est une représentation schématique d'une variante de la partie distale du corps de l'instrument de mesure de la figure 2,
- la figure 5 est un graphe illustrant une fonction de transfert reliant la conductance électrique à la porosité de l'os pour différentes configurations des première et deuxième électrodes,
- les figures 6a et 6b sont des graphes illustrant l'évolution de la conductivité électrique au cours du temps lors d'un forage respectivement dans deux structures osseuses de qualités différentes,

- la figure 7 est une représentation d'un instrument de mesure mis en oeuvre dans un système de détermination de la qualité d'une structure osseuse selon un deuxième mode de réalisation de l'invention,
- les figures 8a et 8b sont des représentations schématiques d'un premier mode de réalisation de système de consolidation d'une structure osseuse mettant en oeuvre un système de détermination de la qualité d'une structure osseuse, et un dispositif de fixation sous la forme d'une cage pour fusion intervertébrale, l'ensemble du système de détermination pouvant être implanté,
- la figure 9 est une représentation schématique d'un système de consolidation d'une structure osseuse selon une variante du premier mode de réalisation des figures 8a et 8b, le dispositif de fixation se présentant sous la forme d'une plaque d'ostéosynthèse,
- la figure 10 est une représentation schématique d'un deuxième mode de réalisation de système de consolidation d'une structure osseuse mettant en oeuvre un système de détermination de la qualité d'une structure osseuse, et un dispositif de fixation sous la forme d'un dispositif fixateur externe.

Sur les figures, les mêmes références désignent des éléments identiques ou analogues.

Les figures représentent un système 1 de détermination de la qualité d'une structure osseuse 2 du corps d'un patient destiné à permettre à un chirurgien d'obtenir une information en temps réel et locale sur la qualité de la structure osseuse 2, et notamment sur sa porosité. Comme il apparaîtra dans la suite de la description, ce système 1 trouve des applications avantageuses non seulement lors d'une intervention chirurgicale sur la structure osseuse 2, par exemple pour la mise en place d'un implant, mais également après l'intervention chirurgicale pour le suivi de la fusion, c'est-à-dire la reconstruction, osseuse et, éventuellement, l'adaptation du traitement de consolidation à l'évolution de cette reconstruction ou l'ajustement d'un programme de rééducation fonctionnelle. Bien que décrite en relation avec une application au corps d'un patient, l'invention s'applique également à la détermination de la qualité de la structure osseuse du corps de tout autre type de sujet et notamment d'un animal ou d'un cadavre.

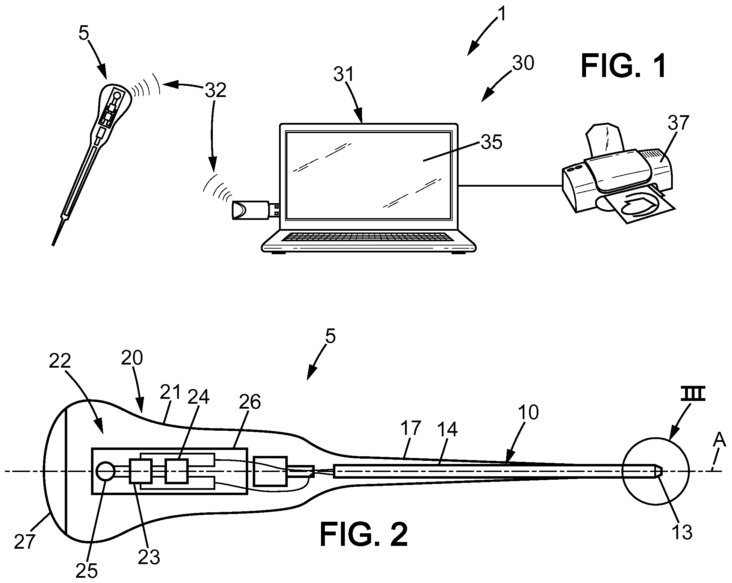

Dans un premier mode de réalisation représenté sur les figures 1 à 6, le système 1 comprend un instrument de mesure 5 et une unité centrale de commande 30.

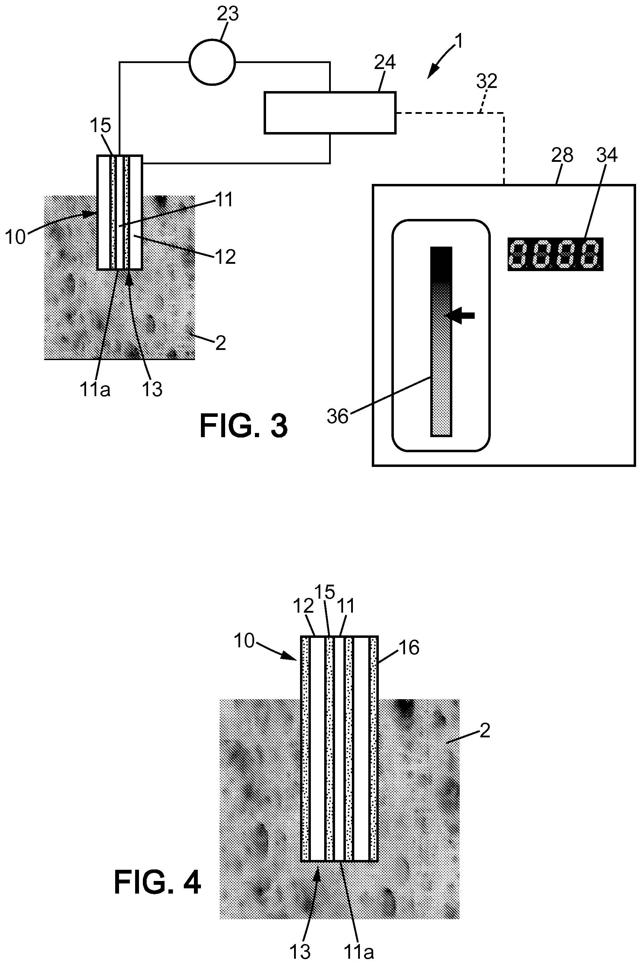

Sur les figures 2 et 3, l'instrument de mesure 5 est un outil à main, tel qu'un outil de forage de la structure osseuse 2 du type de celui décrit dans la demande de brevet WO 03/068076 et connu sous le nom de Pedi-Guard ®.

L'instrument de mesure 5 comprend un corps 10 et un boîtier 20 formant une poignée 21 solidarisée au corps 10.

Bien que décrite en relation avec un instrument de mesure à main adapté au forage de la structure osseuse 2, l'invention n'est pas limitée à ce type d'instrument ni à la réalisation sous la forme d'un instrument comme il apparaîtra dans la suite de la description. En particulier, l'invention peut être mise en oeuvre dans d'autres types d'instruments, notamment une sonde, une pointe carrée, une spatule, une curette ou autre, ou dans un implant comme décrit plus loin.

Le corps 10 présente une surface extérieure 14 et sert de support à des première 11 et deuxième 12 électrodes présentant respectivement des surfaces de contact agencées pour venir en contact avec la structure osseuse 2 à distance l'une de l'autre.

Dans le mode de réalisation représenté, le corps 10, adapté pour le forage de la structure osseuse 2, notamment d'un pédicule vertébral, est cylindrique de section circulaire selon un axe central A et s'étend depuis la poignée 21 jusqu'à une extrémité distale 13. Le corps pourrait, toutefois, présenter toute autre forme, notamment cylindrique de section polygonale ou autre.

Comme représenté schématiquement sur la figure 3 qui illustre une partie distale du corps 10 au voisinage de son extrémité distale 13, le corps 10 réalisé en un matériau conducteur est pourvu d'un alésage central.

La première électrode 11 en matériau conducteur est disposée dans l'alésage central de sorte à s'étendre à l'intérieur du corps 10 parallèlement à l'axe central A, coaxialement à celui-ci, jusqu'à une extrémité libre 11a présentant une surface de contact affleurant la surface extérieure 14 du corps 10 à l'extrémité distale 13. Une couche de matière isolante 15 recouvre une surface intérieure de l'alésage central du corps 10 de sorte à entourer la première électrode 11. De cette manière, la première électrode 11 présente une surface de contact ponctuelle, c'est-à-dire que seule la surface de contact de la première électrode 11 affleure la surface extérieure 14 du corps 12. Pour s'assurer du contact de la première électrode 11 avec la structure osseuse 2, la première électrode 11 présente une dimension supérieure à celle du pore de la structure osseuse qui est généralement comprise entre 100 à 400 $\mu$m.

La deuxième électrode 12 est formée par le corps 10 lui-même et présente une surface de contact composée d'une partie cylindrique parallèle à l'axe centrale A correspondant à une surface latérale du corps 10, et une partie annulaire généralement perpendiculaire à l'axe central A correspondant à une surface distale du corps 10. La deuxième électrode 12 est alors séparée de la première électrode 11 par la couche de matière isolante 15.

De cette manière, la première électrode 11 présente une surface de contact constante vis-à-vis de la structure osseuse à investiguer. Par ailleurs, les première 11 et deuxième 12 électrodes sont placées sur le corps 10 à une distance fixe l'une de l'autre.

L'invention n'est toutefois pas limitée à la réalisation et à l'agencement précédemment décrits du corps et des première 11 et deuxième 12 électrodes. Par exemple, dans une variante représentée sur la figure 4, la deuxième électrode 12 est recouverte sur une surface latérale parallèle à l'axe central A d'une couche de matière isolante 16, de sorte que la surface de contact de la deuxième électrode 12 se limite à la seule la partie annulaire affleurant la surface extérieure 14 du corps 10 à l'extrémité distale 13. Le corps 10 peut alors être réalisé dans la matière isolante, les première 11 et deuxième 12 électrodes étant emmanchées dans le corps 14.

De manière plus générale, les première 11 et deuxième 12 électrodes ne sont pas nécessairement agencées de manière coaxiale. En particulier, ces électrodes peuvent chacune être réalisées par une tige plongée dans le corps 10. Par ailleurs, la première électrode 11 et la deuxième électrode 12 peuvent chacune présenter une surface de contact ponctuelle affleurant la surface latérale ou la surface distale du corps, la deuxième électrode 12 présentant, par exemple, une dimension supérieure à 400 $\mu$m. On peut également prévoir que le corps supporte deux ou plus de deux premières électrodes 11 et deux ou plus de deux deuxièmes électrodes 12.

La poignée 21, cylindrique de révolution, s'étend sensiblement coaxialement à l'axe central A du corps 10. La poignée 21 présente une forme facilitant la prise en main et la manipulation de l'instrument 5. La poignée 21 réalisée en matériau plastique est solidaire d'un manchon 17 de matière plastique s'étendant sur une partie de la surface extérieure 14 du corps 10.

La poignée 21 comporte un logement 22 adapté pour recevoir un générateur électrique 23, un dispositif de mesure 24 et un dispositif d'alimentation 25 alimentant en énergie électrique le générateur électrique 23 et le dispositif de mesure 24. Le générateur électrique 23, le dispositif de mesure 24 et le dispositif d'alimentation 25 sont par exemple placés sur une carte électronique 26 insérée dans le logement 22 au travers d'une ouverture prévue à une extrémité de la poignée 21 opposée au corps 10. Un capot 27 amovible permet de fermer le logement 22.

Les première 11 et deuxième 12 électrodes sont connectées électriquement au générateur électrique 23. Le générateur électrique 23 est adapté pour appliquer sur une durée déterminée un courant électrique entre les surfaces de contact des première 11 et deuxième 12 électrodes. La durée en question peut être variable en fonction de l'application du système de détermination. Il s'agit de la durée au cours de laquelle la qualité de la structure osseuse doit être déterminée, cette durée pouvant comprendre une ou plusieurs mesures en vue de la détermination d'une ou plusieurs valeurs correspondant à la qualité de la structure osseuse comme il apparaîtra dans la suite de la description. En particulier, dans l'application du premier mode de réalisation à un instrument de mesure, la durée peut correspondre à toute la durée de l'intervention chirurgicale ou à une partie de celle-ci, et notamment à la durée du forage. Dans une application à un implant comme évoqué précédemment et décrit plus loin en relation avec un deuxième mode de réalisation de l'invention, la durée peut correspondre à la toute la durée de l'implantation dans le corps du patient ou à une partie de celle-ci.

Il s'agit, en particulier, d'un générateur de tension adapté pour appliquer une tension électrique dont la valeur est connue ou d'un générateur de courant adapté pour appliquer un courant électrique dont l'intensité est connue.

Avantageusement, pour éviter la polarisation des électrodes et donc une perte de précision de la mesure par l'instrument de mesure 5, le générateur électrique 23 est adapté pour appliquer le courant électrique sous la forme d'une impulsion électrique ou d'une série de plusieurs impulsions électriques. Les impulsions électriques de tension ou de courant, par exemple toutes positives, présentent respectivement des intervalles de temps déterminés identiques ou différents les uns des autres. En variante, le générateur électrique pourrait délivrer un courant alternatif, présentant une fréquence quelconque. Bien qu'un courant électrique sous forme d'impulsions ou un courant alternatif soit préféré, on pourrait prévoir que le générateur électrique 23 délivre un courant continu.

Dans un exemple particulier, le générateur électrique est un générateur de tension délivrant des impulsions électriques de tension inférieure à 4 V pour ne pas endommager les nerfs en cas de contact, à une fréquence inférieure à 10 Hz pour permettre une stimulation des muscles sans fibrillation.

Le dispositif de mesure 24 connecté électriquement aux première 11 et deuxième 12 électrodes est adapté pour mesurer en continu, sur la durée déterminée pendant laquelle le générateur électrique 23 applique le courant électrique :

- la tension du courant électrique traversant la structure osseuse 2 entre les surfaces de contact des première 11 et deuxième 12 électrodes, lorsque le générateur électrique 23 est un générateur de courant, ou
- l'intensité du courant électrique traversant la structure osseuse 2 entre les surfaces de contact des première 11 et deuxième 12 électrodes, lorsque le générateur électrique 23 est un générateur de tension.

Le dispositif de mesure 24 est constitué par exemple par un ampèremètre, un voltmètre ou tout autre dispositif de mesure de la tension ou du courant (oscilloscope ou carte électronique dédiée).

Dans l'exemple particulier décrit, le dispositif de mesure mesure l'intensité du courant électrique.

Ainsi, comme représenté sur la figure 3, au cours du forage et de la pénétration du corps 10 dans la structure osseuse 2, les surfaces de contact des première 11 et deuxième 12 électrodes sont en contact avec la structure osseuse 2. La tension électrique connue est appliquée par le générateur électrique 23 entre la surface de contact de la première électrode 11, dont la dimension ne varie pas au cours de la pénétration, et la surface de contact de la deuxième électrode 12 placée à une distance connue de la première électrode 11. L'intensité du courant qui circule dans la structure osseuse 2 entre les surfaces de contact des première 11 et deuxième 12 électrodes est mesurée. Comme indiqué précédemment, on pourrait appliquer un courant d'intensité connue et mesurer la tension.

A partir de la mesure réalisée par l'instrument de mesure 5, une grandeur électrique représentative de l'aptitude de la structure osseuse 2 à laisser passer le courant électrique peut être déterminée et une indication de la qualité de la structure osseuse 2 en contact avec les première 11 et deuxième 12 électrodes peut être déduite.

Pour ce faire, le dispositif de mesure 24 communique, par l'intermédiaire d'une interface de communication 32 par exemple à distance comme représenté sur les figures 1 et 3, avec l'unité centrale de commande 30 qui comporte un dispositif de traitement 28. L'interface de communication 32 peut comporter un premier émetteur/récepteur placé dans l'instrument de mesure 5, par exemple sur la carte électronique 26, et un deuxième émetteur/récepteur placé sur un ordinateur 31 constituant l'unité centrale de commande 30. En variante, la liaison entre l'instrument de mesure 5 et l'unité centrale de commande 30 pourrait être filaire. Il est à noter, par ailleurs, qu'on pourrait prévoir d'intégrer le dispositif de traitement 28 à l'instrument de mesure 5, en le plaçant sur la carte électronique 26.

Le dispositif de traitement 28 est adapté pour déterminer la grandeur électrique précitée, en continu sur la durée déterminée, pendant laquelle le générateur de tension 23 applique le courant électrique à une tension connue et le dispositif de mesure 24 mesure l'intensité du courant traversant la structure osseuse entre les surface de contact des première 11 et deuxième 12 électrodes. En particulier, le dispositif de traitement 28 est adapté pour calculer en continu pendant la durée déterminée une valeur pour la grandeur électrique et pour associer en continu pendant la durée déterminée la valeur calculée pour la grandeur électrique à une valeur pour la qualité de la structure osseuse 2, notamment de la porosité de la structure osseuse 2. Le dispositif de traitement 28 est, par ailleurs, adapté pour délivrer en continu sur la durée déterminée un signal représentatif de la qualité de la structure osseuse, notamment de la porosité de la structure osseuse 2, entre les surfaces de contact des première 11 et deuxième 12 électrodes à partir de la grandeur électrique déterminée.

Dans l'exemple particulier décrit, la grandeur électrique est la conductivité de la structure osseuse 2 entre les surfaces de contact des première 11 et deuxième 12 électrodes.

En effet, la conductivité électrique est une propriété intrinsèque du matériau qui dépend de la nature du matériau. La structure osseuse 2 étant composée de deux phases, à savoir de l'os solide peu conducteur et de la moelle osseuse contenant des cellules sanguines conductrice, ayant des conductivités électriques distinctes, la mesure de la conductivité électrique de la structure osseuse 2 permet d'obtenir une information sur la proportion de ces deux phases et donc sur la qualité de la structure osseuse 2. En effet, dans un os très dur comme l'os cortical, la conductivité électrique sera faible parce que la phase solide, peu conductrice, sera prépondérante alors que la conductivité électrique sera plus élevée dans un os ostéoporotique étant donné une plus forte présence de sang, très conducteur. Ainsi, plus la conductivité électrique sera élevée et moins la structure osseuse sera de bonne qualité.

Il apparaît de ce qui précède que la porosité représentative de la proportion de chacune des phases composant la structure osseuse 2 constitue un indicateur de la qualité de la structure osseuse 2. On peut toutefois prévoir d'utiliser d'autres indicateurs de la qualité de la structure osseuse 2 basé sur la proportion de chacune des phases composant la structure osseuse 2. Par exemple, la proportion de masse osseuse M, avec M = 1 - P où P est la porosité de la structure osseuse, pourrait servir d'indicateur de la qualité de la structure osseuse 2.

Comme indiqué précédemment, pour mesurer la capacité d'un matériau à laisser passer le courant on applique la tension connue, en Volts, entre les surfaces de contact des première 11 et deuxième 12 électrodes 2 en contact avec la structure osseuse 2 et on mesure l'intensité, en Ampère, qui en résulte.

Le dispositif de traitement 28 peut alors établir un premier rapport de l'intensité, mesurée dans l'exemple considérée, sur la tension, connue dans l'exemple considéré. Ce premier rapport correspond à la conductance mesurée en Siemens (S).

La conductivité électrique σ (en S/m), caractéristique du matériau investigué, s'exprime de la façon suivante :

$$\sigma = \frac{L \times I}{A \times V}$$

où I (en A) est l'intensité du courant électrique entre les surfaces de contact des première 11 et deuxième 12 électrodes, mesurée dans l'exemple considéré mais qui peut être connue,
V (en V) est la tension du courant électrique entre les surfaces de contact des première 11 et deuxième 12 électrodes, connue dans l'exemple considéré mais qui peut être mesurée,
L (en m) est la distance entre les surfaces de contact des première 11 et deuxième 12 électrodes,
A (en m$^2$) représente les dimensions des surfaces de contact des première 11 et deuxième 12 électrodes avec le matériau.

Le système de détermination pouvant être intégré à tout type d'instrument chirurgical, de nombreuses configurations, notamment en termes d'agencement et de géométrie, pour les première 11 et deuxième 12 électrodes peuvent être utilisées. Pour chaque géométrie, il faut alors déterminer un facteur de calibration correspondant à un deuxième rapport entre la distance L entre les surfaces de contact des première 11 et deuxième 12 électrodes et les dimensions A des surfaces de contact des première 11 et deuxième 12 électrodes avec la structure osseuse.

Lorsque, comme dans le cas présent, les surfaces de contact des première 11 et deuxième 12 électrodes n'ont pas les mêmes dimensions, la valeur prise en compte pour la dimension A des surfaces de contact des première 11 et deuxième 12 électrodes avec le matériau est la dimension de la plus petite surface de contact. La surface de contact de la deuxième électrode 12 augmentant au fur et à mesure de la pénétration de l'instrument de mesure 5 dans la structure osseuse 2, la plus petite surface de contact à prendre en compte est celle de la première électrode 11, qui reste constante au fur et à mesure de la pénétration.

Ainsi, dans le mode de réalisation représenté, connaissant l'agencement et la géométrie des surfaces de contact des première 11 et deuxième 12 électrodes, la calibration pourra être effectuée lors de la conception de l'instrument de mesure 5 et le facteur de calibration déterminé une fois pour toute. Pour cela, le premier rapport entre l'intensité et la tension sera mesuré pour une qualité d'os connue et, connaissant la conductivité électrique de la phase conductrice, le facteur de calibration pourra être déduit. En particulier, il suffit de mesurer la valeur de conductance dans la phase conductrice de la structure osseuse (sang) et dire que cela correspond à 100% de porosité. Pour la phase isolante de la structure osseuse (os solide), la conductance est de 0 pour une porosité de 0%.

Le dispositif de traitement 28 peut comporter une mémoire dans laquelle sont mémorisés le facteur de calibration et/ou la distance L entre les surfaces de contact des première 11 et deuxième 12 électrodes et les dimensions A des surfaces de contact des première 11 et deuxième 12 électrodes avec la structure osseuse. L'utilisateur de l'instrument de mesure 5 n'a alors plus à se soucier de cet aspect. Dans le mode de réalisation représenté, le facteur de calibration est dans la plage 380-500 en m/m2.

Le système décrit précédemment, dans lequel le facteur de calibration est mémorisé, permet donc de mesurer la conductivité électrique de la structure osseuse 2 à partir d'une simple mesure d'intensité (ou de tension).

En variante, lorsque les surfaces de contact des première 11 et deuxième 12 électrodes varient toutes deux, on peut prévoir que le dispositif de traitement 28 soit adapté pour :

- déterminer la distance L entre les surfaces de contact des première 11 et deuxième 12 électrodes,
- déterminer des dimensions A des surfaces de contact des première 11 et deuxième 12 électrodes avec la structure osseuse 2, et
- établir le facteur de calibration.

Comme il apparaît de ce qui a été décrit précédemment, pour un même agencement et une même géométrie des surfaces de contact des première 11 et deuxième 12 électrodes, la conductance pourra être directement représentative de la qualité de la structure osseuse.

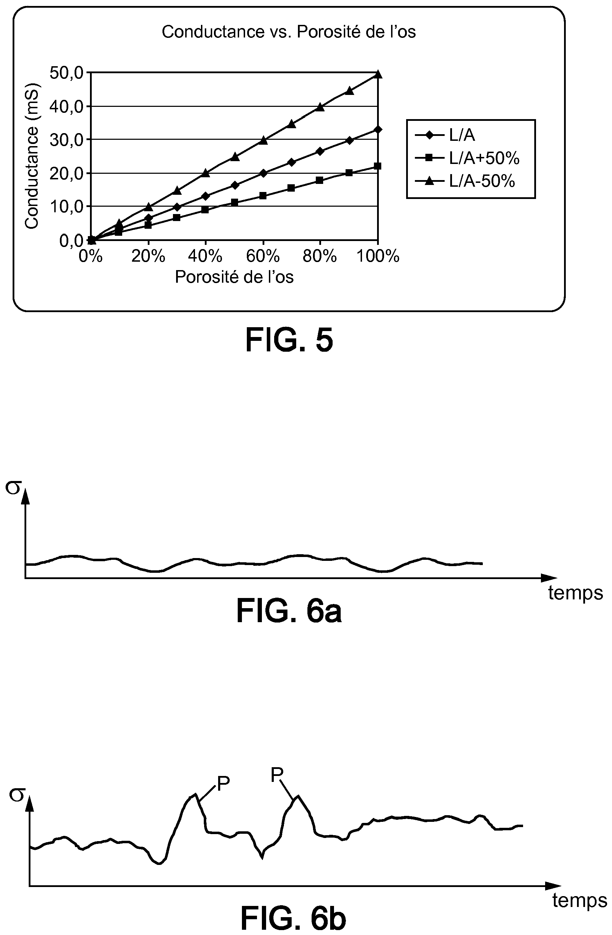

Sur la figure 5, une fonction de transfert reliant la conductance à la porosité de la structure osseuse est représentée. En particulier, la fonction de transfert relie chacune des valeurs d'une plage de valeurs pour la conductance à une valeur d'une plage de valeurs pour la porosité. Une telle fonction de transfert est mémorisée dans la mémoire du dispositif de traitement 28.

La fonction de transfert, par exemple sensiblement linéaire, peut être obtenue par calibration dans un milieu conducteur et un milieu isolant donnant respectivement des valeurs extrêmes pour la grandeur électrique. Les valeurs extrêmes pour la grandeur électrique correspondent alors à des valeurs extrêmes pour la porosité de la structure osseuse. Les valeurs extrêmes pour la grandeur électrique définissent la plage de valeurs pour la grandeur électrique dans laquelle chacune des valeurs pour la grandeur électrique correspond à une valeur pour la porosité de la structure osseuse.

Pour l'instrument de mesure représenté, la plage de mesure du premier rapport entre le tension et l'intensité compris entre 300Ω et 10KΩ en termes d'impédance (rapport

V/I) pour des os de la colonne vertébrale, et entre $1.10^{-4}$ S et $3,3.10^{-3}$ S en terme de conductance (rapport I/V) pour des qualités d'os allant de l'os cortical très dur au sang (cas où il n'y aurait quasiment plus de structure osseuse).

Le graphe de la figure 5 représente la relation entre la mesure de conductance et la porosité de l'os obtenue avec le facteur L/A, correspondant à l'agencement et la géométrie précédemment décrits de l'instrument de mesure 5. Il a été rajouté sur ce graphe les courbes que l'on obtient avec un facteur de calibration L/A 50 % plus grand et un facteur de calibration L/A 50 % plus petit que celui précédemment décrit. Les valeurs de l'abscisse sont données en % de la porosité de la structure osseuse, ce qui correspond à la quantité de sang présent dans la structure osseuse (0% étant un matériau complètement isolant et 100% la mesure obtenue dans du sang pur).

La description qui vient d'être faite du dispositif de traitement 28 exploitant la mesure de la conductance et la détermination de la conductivité de la structure osseuse est directement transposable à un dispositif de traitement 28 exploitant la mesure de l'impédance et la détermination de la résistivité de la structure osseuse.

Ainsi, dans le système de détermination selon l'invention, dès qu'une intensité (respectivement une tension) est mesurée, la tension (respectivement l'intensité) étant connue, la valeur de la conductance (ou de l'impédance) est déterminée. Cette valeur de la conductance (ou de l'impédance) correspond, directement ou indirectement via le facteur de calibration qui permet d'obtenir la valeur de la conductivité (ou de la résistivité), à la valeur de la porosité de la structure osseuse 2 et donc une valeur de sa qualité.

Le dispositif de traitement 28 peut délivrer en continu pendant la durée déterminée le signal représentatif de la qualité de la structure osseuse entre les surfaces de contact des première et deuxième électrodes.

Ce signal peut être de toute nature appropriée, électrique, visuel, auditif ou autre. Il peut notamment s'agir d'un signal électrique communiqué à l'unité centrale de commande 30 pour un traitement ultérieur. Le dispositif de traitement 28 peut également comporter un indicateur de résultat pour la qualité de la structure osseuse. L'indicateur de résultat peut se présenter sous la forme de tout dispositif permettant de donner une indication visuelle de la qualité de la structure osseuse. En particulier, l'indicateur de résultat, tel qu'un afficheur 34, un écran d'ordinateur 35, un galvanomètre, une échelle graduée lumineuse 36, par exemple une barre de LED, ou autre, peut permettre de lire une valeur déterminée de la qualité de la structure osseuse. En combinaison ou de façon alternative à l'indication visuelle prévue précédemment, l'indicateur de résultat pourrait fournir une indication sonore. Le dispositif de traitement 28 peut être connecté à un dispositif de sauvegarde et/ou de documentation des résultats obtenus, sous la forme, dans le mode de réalisation représenté d'un enregistrement numérique, notamment sur le disque dur de l'ordinateur 31, et une imprimante 37, afin de donner la possibilité au spécialiste d'établir un diagnostic.

Outre l'indication instantanée de la qualité locale de la structure osseuse, on peut prévoir d'enregistrer, par exemple dans la mémoire du dispositif de traitement 28, l'ensemble des valeurs représentatives de la qualité de la structure osseuse sur la durée déterminée.

Par exemple, sur les figures 6a et 6b, chacune des valeurs de la conductivité représentative de la qualité locale de la structure osseuse 2 mesurée sur la durée déterminée est, d'une part, mesurée et indiquée instantanément et, d'autre part, sommée aux autres valeurs mesurées sur la durée déterminée pour permettre une intégration mathématique des valeurs de la conductivité. L'intégration mathématique représentative de l'ensemble du forage peut alors donner une indication de la qualité globale du forage, en tenant compte des brèches effectuées au cours du trajet de l'instrument de mesure 5 et des inhomogénéités rencontrées. En particulier, on peut prévoir que l'ancrage d'un implant sera de meilleure qualité dans le forage dont la mesure des valeurs de la conductivité est représentée sur la figure 6a que dans celui dans le forage dont la mesure des valeurs de la conductivité est représentée sur la figure 6b. En effet, cette dernière mesure fait apparaître deux pics P correspondant à des brèches dans la structure osseuse 2.

Ainsi, le système de détermination 1 permet une mesure précise, locale et instantanée donnant une information immédiate au cours de l'intervention chirurgicale sur la qualité de la structure osseuse sur laquelle le chirurgien intervient directement. Comme il est apparu, ce système est aisément intégrable dans un instrument chirurgical. La qualité de la structure osseuse peut donc être déterminée sans allonger le temps de l'intervention chirurgicale.

L'information obtenue concernant la qualité de la structure osseuse 2 permet d'éclairer le chirurgien dans ses décisions thérapeutiques pendant et après l'intervention et de l'influencer sur le meilleur choix pour son patient. Par exemple, lors de l'intervention, le chirurgien pourra optimiser :

- le type d'implant à mettre en œuvre (vis à expansion), ses dimensions, (diamètre plus ou moins gros), la mise en place d'un crochet, etc.,
- le nombre d'implant : ajout d'un niveau (une vertèbre supplémentaire), décision de ne pas placer de vis dans un os trop fragile, etc.,
- les traitements complémentaires : mise en place ou non d'un ciment de consolidation, etc.,
- le placement de l'implant : placement d'un implant de façon optimale en choisissant le lieu exact d'implantation connaissant la solidité de l'os localement (ex: sacrum bicortical), etc.,
- le type de manœuvre à effectuer pour ajuster les contraintes mécaniques per-opératoires à la qualité de l'os, notamment les manœuvres de correction de déformation, ou de spondylolisthesis, qui pourraient entraîner si elles sont trop puissantes, des arrachements per-opératoires d'implants, etc.

Le système de détermination selon invention s'applique également à la mesure continue de la reconsolidation osseuse après fracture ou après fusion.

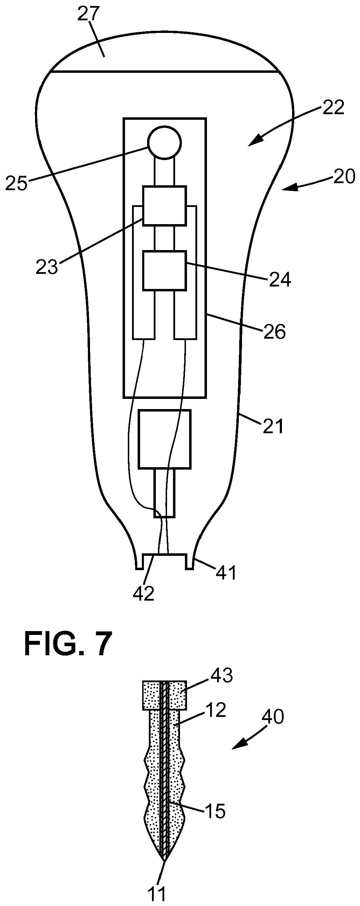

A cet égard, on décrit en relation avec la figure 7 un deuxième mode de réalisation du système de détermination de la qualité de la structure osseuse. Ce deuxième mode de réalisation ne diffère du premier mode, dont il reprend les principaux composants, qu'en ce que la poignée 21 et le corps 40 sont solidarisés de manière amovible. On se réfèrera à la description précédemment faite pour plus de détails sur les composants.

Le corps 40 se présente par exemple sous la forme d'une vis, notamment une vis pédiculaire, destinée à être implantée dans une structure osseuse 2. Le corps est réalisé en un matériau biocompatible, implantable dans la structure osseuse 2 et présente une surface extérieure adaptée pour permettre l'ancrage du corps dans la structure osseuse 2. En particulier, dans le cas d'une vis, la surface extérieure est pourvue d'un filetage. Comme pour le corps 10 précédemment décrit, le corps 40 porte les première 11 et deuxième 12 électrodes agencées de manière analogue à celle précédemment décrite, sans toutefois y être limité.

La poignée 21 présente une extrémité 41 adaptée pour engager de manière amovible le corps 40 et l'entraîner en rotation afin de le faire pénétrer dans la structure osseuse. En particulier, un logement 42, par exemple de forme polygonal, adapté pour recevoir une tête 43 solidaire du corps 40, par exemple de forme polygonal analogue à celle du logement 42, peut être prévue. En variante, la poignée 42 pourrait être conformée comme un tournevis avec une extrémité adaptée pour engager une fente sur la tête 43.

L'extrémité de la poignée 21 comporte alors des organes de connexion électrique, tels que des contacts électriques, agencés pour venir en contact avec les première 11 et deuxième 12 électrodes lorsque le corps 40 est solidarisé à la poignée, afin de connecter électriquement les première 11 et deuxième 12 électrodes au générateur électrique 23 et au dispositif de mesure 24.

Le système de détermination selon le deuxième mode de réalisation peut avantageusement être utilisé dans un système de consolidation de la structure osseuse dans lequel le générateur électrique 23 est adapté pour appliquer un signal électrique de stimulation de croissance osseuse. Cette disposition permet de mesurer l'évolution de la croissance osseuse, par la mesure de l'évolution de la qualité de la structure osseuse, et d'utiliser cette information comme donnée d'entrée du générateur électrique 23 en tant que stimulateur électrique, afin d'optimiser le signal électrique de stimulation émis en fonction de la valeur obtenue de la qualité de la structure osseuse.

L'optimisation du signal électrique de stimulation peut être réalisée de manière manuelle, par le chirurgien auquel la qualité de la structure osseuse a été communiquée par tout moyen approprié. En variante, l'optimisation du signal électrique de stimulation peut être réalisée de manière automatique. On peut alors prévoir, pour ce faire, que l'unité centrale de commande 30, par le biais du dispositif de traitement 28, soit adaptée pour commander le générateur électrique 23 en fonction du signal représentatif de la qualité de la structure osseuse 2, notamment de la porosité de la structure osseuse. Les traitements de consolidation peuvent ainsi être adaptés automatiquement par le biais d'une boucle de rétroaction.

L'utilisation d'une telle boucle de rétroaction pour commander le générateur électrique et le dispositif de mesure n'est pas limitée à l'application précitée à la consolidation de la structure osseuse par une stimulation électrique.

On peut, en outre, prévoir que le système de détermination de la qualité de la structure osseuse soit combiné, dans un autre système de consolidation, à tout dispositif de stimulation électrique autre que celui constitué par le générateur électrique 23 et les première 11 et deuxième 12 électrodes. Il suffit pour cela d'utiliser tout dispositif de stimulation électrique approprié permettant d'appliquer une stimulation électrique de croissance osseuse sur la structure osseuse. De préférence, la stimulation électrique est réglable pour pouvoir l'ajuster, manuellement ou automatiquement, en fonction du signal représentatif de la qualité de la structure osseuse.

Par ailleurs, le système de consolidation de la structure osseuse n'est pas limité à la mise en œuvre du système de détermination de la qualité osseuse selon le deuxième mode de réalisation. En particulier, le système de détermination de la qualité osseuse selon le premier mode de réalisation ou tout autre système de détermination de la qualité osseuse fonctionnant selon le principe de détermination décrit précédemment pourrait être mis en œuvre dans le système de consolidation.

Par exemple, dans le cadre d'une application à la fusion osseuse mais également de toute autre application, puisque la mesure de la conductivité électrique permet de connaître la qualité de la structure osseuse environnante, les première 11 et deuxième 12 électrodes, le générateur de tension ou de courant 23, le dispositif de mesure 24, le dispositif d'alimentation 25 ainsi qu'une interface de communication à distance avec le dispositif de traitement 28 pourraient être intégrés dans un implant (vis, plaque, tige, cage, lead ou autre) placé à l'intérieur du corps du patient, sous la peau. Lors de la guérison du patient le médecin pourra ainsi lire à distance la valeur de la conductivité électrique mesurée et connaître l'état de la consolidation osseuse et de l'ancrage.

Les première 11 et deuxième 12 électrodes peuvent alors être portées par un corps en un matériau biocompatible, implantable dans la structure osseuse, le générateur de tension ou de courant 23, le dispositif de mesure 24, le dispositif d'alimentation 25 et l'interface de communication à distance étant placés dans un boîtier également réalisé en un matériau biocompatible, implantable à l'intérieur du corps du patient. Des organes de fixation permettent d'assurer la fixation du boîtier et du corps au voisinage de la structure osseuse 2, directement sur la structure osseuse et/ou sur des tissus entourant la structure osseuse.

De façon complémentaire ou alternative à la stimulation électrique, le système de consolidation peut comprendre un dispositif de fixation adapté pour immobiliser la structure osseuse de sorte à permettre la reconstruction osseuse. La mesure de la qualité de la structure osseuse permet alors de suivre l'évolution de la reconstruction osseuse autour du dispositif de fixation.

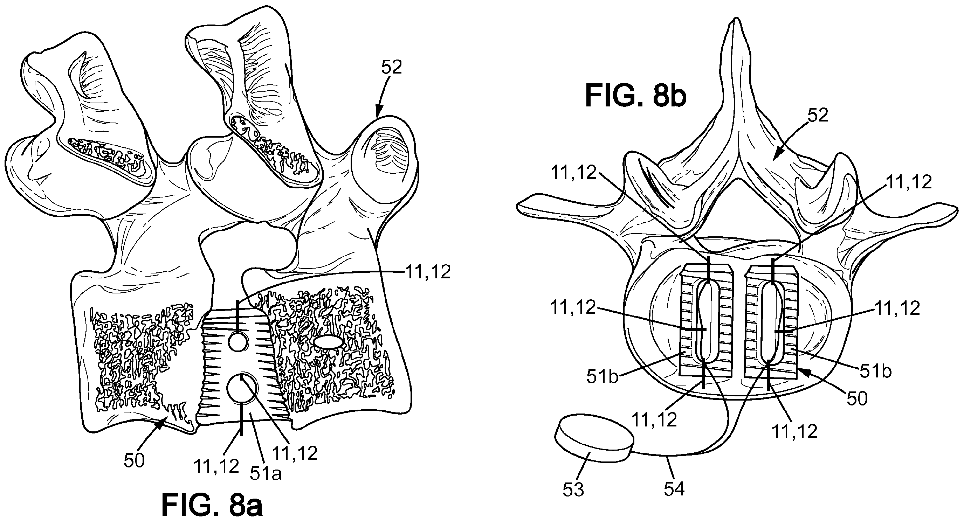

Sur les figures 8a et 8b, on représente de manière schématique un premier mode de réalisation de système de consolidation mettant en œuvre un système de détermination de la qualité d'une structure osseuse, et un dispositif de fixation.

Le dispositif de fixation est réalisé sous la forme d'une cage 50 pour fusion intervertébrale à laquelle sont associées plusieurs électrodes pouvant former tour à tour les première 11 et deuxième 12 électrodes du système de détermination de la qualité de la structure osseuse. En particulier, la cage 50 peut comprendre une partie filetée, tronconique 51a sur la figure 8a et cylindrique 51b sur la figure 8b, implantée dans une vertèbre 52. Les premières 11 et deuxièmes 12 électrodes sont intégrés à la partie filetée 51a de manière à avoir leur surface de contact en contact avec une partie située entre deux vertèbres 52 où un greffon osseux a été déposé en vue d'obtenir une fusion solide des deux vertèbres 52. La partie filetée 51a forme ainsi un corps auquel est connecté un boîtier 53 enfermant au moins le générateur de tension ou de courant 23, le dispositif de mesure 24 et le dispositif d'alimentation 25.

Le boîtier 53 est interne ou implantable, c'est-à-dire placé sous la peau du patient en étant solidarisé, par exemple, aux tissus mous à proximité de la vertèbre 52. Le boîtier 53 est alors connecté aux premières 11 et deuxièmes 12 électrodes par une liaison filaire 54 et comprend une interface de communication, de préférence à distance, pour communiquer avec le dispositif de traitement 28 si celui-ci n'est pas intégré au boîtier 53 ou avec une unité centrale de commande.

On peut toutefois prévoir que le boîtier 53 soit externe, c'est-à-dire placé à l'extérieur du patient et relié par une liaison filaire ou autre aux premières 11 et deuxièmes 12 électrodes. Le boîtier 53 peut alors communiquer avec le dispositif de traitement 28 si celui-ci n'est pas intégré au boîtier 53 ou avec une unité centrale de commande par une interface de communication filaire ou à distance.

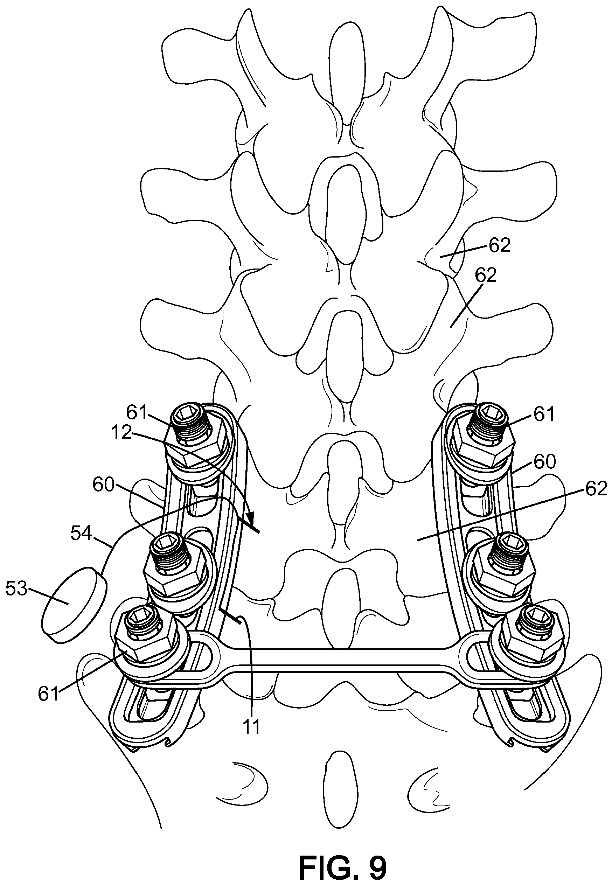

En variante représentée schématiquement sur la figure 9, le système de consolidation comprend un dispositif de fixation sous la forme de deux plaques d'ostéosynthèse 60 fixées, parallèlement l'une à l'autre, à plusieurs vertèbres 62 par l'intermédiaire de vis de fixation 61. Les première 11 et deuxième 12 électrodes sont intégrées au dispositif de fixation, par exemple à l'une des plaques d'ostéosynthèse 60, à chacune des plaques d'ostéosynthèse 60 ou aux vis de fixation 61. Le boîtier 53, interne ou externe, est relié aux première 11 et deuxième 12 électrodes par la liaison filaire 54.

Avantageusement, le dispositif de fixation peut être adapté pour appliquer une contrainte mécanique réglable sur la structure osseuse, de sorte à pouvoir ajuster la contrainte mécanique sur la structure osseuse en fonction du signal représentatif de la qualité de la structure osseuse.

En effet, selon la loi de Wolff une consolidation optimale (d'un point de vue mécanique) de l'os est obtenue quand l'os est soumis lors de sa reconstruction a des contraintes mécaniques qui ne sont ni trop faibles (des contraintes mécaniques trop faibles ne stimulant pas la croissance osseuse) ni trop importantes (des contraintes mécaniques trop importantes pouvant entraîner la rupture des fibres osseuses encore fragiles pendant la reconstruction).

Comme indiqué précédemment, l'ajustement peut être réalisé manuellement par le chirurgien ou automatiquement au moyen d'une boucle de rétroaction dans laquelle le dispositif de traitement commande le dispositif de fixation en fonction du signal représentatif de la qualité de la structure osseuse.

En particulier, le dispositif de fixation peut présenter une géométrie variable permettant de faire varier la contrainte mécanique sur la structure osseuse. Par exemple, le dispositif de fixation peut comprendre au moins deux éléments de fixation implantables dans la structure osseuse à distance l'un de l'autre, et un élément de liaison reliant les éléments de fixation, ledit élément de liaison présentant une rigidité ajustable.

L'élément de liaison peut comprendre une portion élastique conférant une certaine souplesse à la liaison entre les éléments de fixation, et un actionneur piézoélectrique agencé pour ajuster la rigidité de la portion élastique. Par exemple, l'élément de liaison peut comprendre un ressort précontraint dont la précontrainte peut être ajustée par l'action de l'actionneur piézoélectrique activé par une source d'énergie.

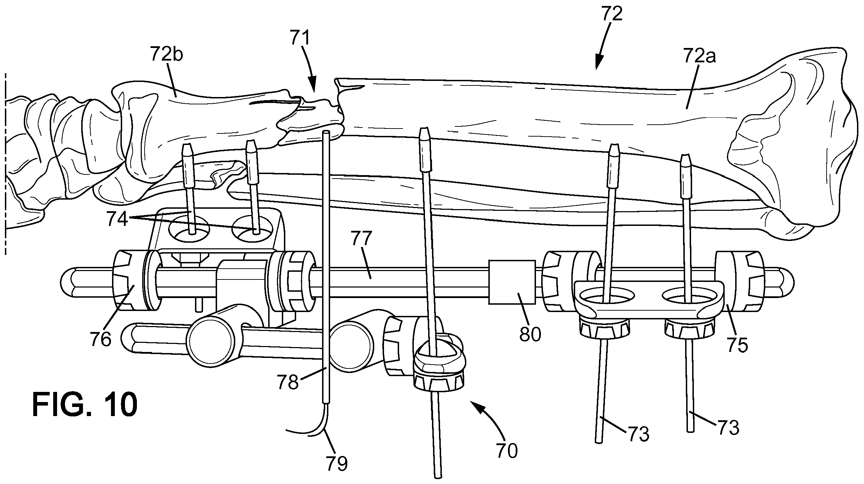

Par exemple, la figure 10 illustre un deuxième mode de réalisation de système de consolidation d'une structure osseuse 72 mettant en œuvre un dispositif de fixation sous la forme d'un dispositif orthopédique de « fixateurs externes » 70, qui permet la consolidation osseuse de fractures en assurant un lien mécanique par un élément de liaison externe entre des broches, comme éléments de fixation, implantées de part et d'autre d'un trait de fracture, et qui se prolongent à l'extérieur du corps du patient.

Sur la figure 10, la structure osseuse 72, ici un tibia, présente une première partie 72a et une deuxième partie 72b placées de part et d'autre d'une fracture 71. Le dispositif orthopédique de « fixateurs externes » 70 comprend un premier ensemble de broches 73 enfoncées dans la première partie 72a du tibia 72, et un deuxième ensemble de broches 74 enfoncées dans la deuxième partie 72b du tibia 72. Les broches 73 du premier ensemble sont portées de manière réglable par un premier chariot 75 et les broches 74 du deuxième ensemble sont portées de manière réglable par un deuxième chariot 76. Les premier 75 et deuxième 76 chariots, placés à l'extérieur du patient, sont montés sur une barre 77, généralement parallèle au tibia 72, de manière coulissante afin de pouvoir faire varier leur écartement.

Une broche de mesure 78, constituant le corps du système de détermination de la qualité de la structure osseuse et sur laquelle sont placées les premières 11 et deuxième 12 électrodes, s'étend depuis l'extérieur du patient jusqu'à la fracture 71 de telle manière que les surfaces de contact des première 11 et deuxième 12 électrodes soient en contact avec la structure osseuse au niveau de la fracture 71. Les première 11 et deuxième 12 électrodes sont reliées au générateur électrique 23, au dispositif de mesure 24 et au dispositif de traitement 28 de toute manière appropriée, par exemple par une liaison filaire 79.

En fonction de la qualité de la structure osseuse déterminée par le système de détermination, l'écartement entre les premier 75 et deuxième 76 chariots peut être réglé, et les contraintes mécaniques exercées sur la structure osseuse ajustées. Ce réglage peut être réalisé manuellement, le chirurgien actionnant directement des organes de réglage, tels que des mollettes, prévus sur les premier 75 et deuxième 76 chariots, ou de façon automatisée, des actionneurs actionnant les organes de réglage.

De façon alternative ou complémentaire au réglage des premier 75 et deuxième 76 chariots, on peut prévoir que la barre 77 ait une portion élastique 80 dont la rigidité peut être ajustée de façon manuelle ou automatique en fonction de la qualité de la structure osseuse mesurée. L'intégration de la détermination de la qualité de la structure osseuse à un tel dispositif de fixateurs externes permet de mesurer l'évolution de la croissance osseuse et d'ajuster la rigidité de l'élément de liaison mécanique entre les broches 73, 74 des premier et deuxième ensembles afin d'optimiser la qualité et la rapidité de la fusion.

Par ailleurs, le dispositif de fixation peut être un dispositif orthopédique de « fixateurs internes » ou un implant d'arthrodèse, qui permet la consolidation osseuse de fractures ou d'arthrodèse, c'est-à-dire une intervention chirurgicale au cours de laquelle une articulation est remplacée par une fusion, généralement à l'aide de greffe osseuse. Un lien mécanique par un élément de liaison interne, par exemple sous la forme d'une plaque ou d'une tige métallique ou en polymère, est assuré entre des vis, comme éléments de fixation, implantées de part et d'autre du trait de fracture ou de l'articulation fusionnée.

L'intégration de la détermination de la qualité de la structure osseuse à un tel dispositif de fixateurs internes permet de mesurer l'évolution de la croissance osseuse, de telle sorte que, après l'intervention, le chirurgien pourra :

- optimiser les traitements de consolidation : corset / stabilisation supplémentaire, fixateurs externes, ajustement (par une opération mineure per-cutanée sous anesthésie locale, par une commande à distance agissant sur l'implant ou de manière entièrement automatisée) de la rigidité de l'implant, stimulation de croissance osseuse, médicaments, etc.,
- optimiser les décisions thérapeutiques : ré-intervention selon la qualité de l'ancrage in-situ postopératoire, traitement de la douleur associée à une pseudarthrose, etc.,
- suivre le patient et lui porter les recommandations optimales pour sa reconstruction osseuse (rééducation, gestes à éviter, etc.).

Il est à noter que le système de détermination décrit ci-dessus, bien que particulièrement intéressant dans des applications per-opératoires et post-opératoires *in vivo,* puisque donnant un résultat instantané de la qualité de la structure osseuse, peut aussi s'appliquer sur un échantillon d'os *in vitro* afin de déterminer sa qualité instantanément sans autre recours. Pour cela il suffit de placer l'échantillon d'os prélevé, par biopsie par exemple, dans une solution conductrice, notamment un sérum physiologique, d'appliquer les première 11 et deuxième 12 électrodes de géométrie connues, de mesurer la conductance et d'en déduire la qualité de la structure osseuse comme précédemment décrit.

## Revendications

1. Système (1) de détermination de la qualité d'une structure osseuse (2) d'un sujet, notamment de la porosité d'une structure osseuse (2), ledit système (1) comprenant :

   - un corps (10 ; 40) adapté pour réaliser un forage de la structure osseuse (2) et présentant une surface extérieure (14),
   - au moins une première électrode (11) présentant une surface de contact agencée sur la surface extérieure (14) du corps (10 ; 40) pour venir en contact avec la structure osseuse (2) lors du forage,
   - au moins une deuxième électrode (12) présentant une surface de contact agencée sur la surface extérieure (14) du corps (10 ; 40) pour venir en contact avec la structure osseuse (2) à distance de la première électrode (11) lors du forage,
   - un générateur électrique (23) adapté pour appliquer pendant une durée déterminée un courant électrique entre les surfaces de contact des première (11) et deuxième (12) électrodes, ledit courant électrique présentant une caractéristique choisie parmi une tension et une intensité qui est connue,
   - un dispositif de mesure (24) adapté pour mesurer en continu pendant la durée déterminée l'autre caractéristique choisie parmi la tension et l'intensité du courant électrique traversant la structure osseuse (2) entre les surfaces de contact des première (11) et deuxième (12) électrodes,
   - un dispositif de traitement (28) adapté pour déterminer en continu pendant la durée déterminée une grandeur électrique représentative de l'aptitude de la structure osseuse (2) à laisser passer le courant électrique entre les surfaces de contact des première (11) et deuxième (12) électrodes à partir de la caractéristique connue et de la caractéristique mesurée, et pour délivrer en continu pendant la durée déterminée un signal représentatif de la qualité de la structure osseuse (2), notamment de la porosité de la structure osseuse (2), entre les surfaces de contact des première (11) et deuxième (12) électrodes à partir de la grandeur électrique déterminée, **caractérisé en ce que** le dispositif de traitement (28) est adapté pour calculer en continu pendant la durée déterminée une valeur pour la grandeur électrique et pour associer en continu pendant la durée déterminée la valeur calculée pour la grandeur électrique à une valeur pour la qualité de la structure osseuse, notamment pour la porosité de la structure osseuse (2).

2. Système (1) selon la revendication 1, dans lequel le générateur électrique (23) est adapté pour appliquer le courant électrique sous la forme d'au moins une impulsion électrique.

3. Système (1) selon la revendication 1 ou 2, dans lequel le dispositif de traitement (28) est adapté pour mémoriser une fonction de transfert reliant chacune des valeurs d'une plage de valeurs pour la grandeur électrique à une valeur d'une plage de valeurs pour la qualité de la structure osseuse (2), notamment pour la porosité de la structure osseuse (2).

4. Système (1) selon l'une quelconque des revendications 1 à 3, dans lequel le dispositif de traitement (28) est adapté pour établir un premier rapport entre la caractéristique connue et la caractéristique mesurée, et pour déterminer la grandeur électrique à partir dudit premier rapport.

5. Système selon la revendication 4, dans lequel la grandeur électrique est choisie parmi la conductivité et la résistivité de la structure osseuse (2) entre les surfaces de contact des première (11) et deuxième (12) électrodes, le dispositif de traitement (28) étant adapté pour :

   - déterminer une distance entre les surfaces de contact des première (11) et deuxième (12) électrodes,
   - déterminer des dimensions des surfaces de contact des première (11) et deuxième (12) électrodes avec la structure osseuse (2),
   - établir un deuxième rapport entre ladite distance et lesdites dimensions, et
   - calculer la grandeur électrique à partir des premier et deuxième rapports.

6. Système (1) selon la revendication 5, dans lequel le dispositif de traitement (28) est adapté pour mémoriser la distance entre les surfaces de contact des première (11) et deuxième (12) électrodes, et les dimensions des surfaces de contact des première (11) et deuxième (12) électrodes avec la structure osseuse (2).

**7.** Système (1) selon l'une quelconque des revendications 1 à 6, dans lequel la surface de contact d'au moins l'une des première (11) et deuxième (12) électrodes présente une dimension supérieure à 400 μm.

**8.** Système (1) selon l'une quelconque des revendications 1 à 7, dans lequel le dispositif de traitement (28) est adapté pour mémoriser un ensemble de valeurs pour la grandeur électrique sur la durée déterminée.

**9.** Système (1) selon l'une quelconque des revendications 1 à 8, dans lequel le corps (10 ; 40) s'étend selon un axe central (A) jusqu'à une extrémité distale (13), au moins l'une des première (11) et deuxième (12) électrodes s'étendant parallèlement à l'axe central (A), la surface de contact de ladite électrode affleurant la surface extérieure (14) du corps (10 ; 40) à l'extrémité distale (13).

**10.** Système (1) selon l'une quelconque des revendications 1 à 9, dans lequel au moins l'une des première (11) et deuxième (12) électrodes s'étend à l'intérieur du corps (10 ; 40) jusqu'à une extrémité libre présentant la surface de contact, ledit corps (10 ; 40) comportant une couche de matière isolante (15) entourant ladite électrode de telle sorte que seule ladite surface de contact affleure la surface extérieure (14) du corps (10 ; 40).

**11.** Système (1) selon l'une quelconque des revendications 1 à 10, comprenant un boîtier (21) solidarisé au corps (10 ; 40) et un dispositif d'alimentation (25) alimentant en énergie électrique au moins le générateur électrique (23) et le dispositif de mesure (24), ledit boîtier (21) étant adapté pour recevoir au moins le générateur électrique (23), le dispositif de mesure (24) et le dispositif d'alimentation (25).

**12.** Système (1) selon la revendication 11, dans lequel le boîtier (21) forme une poignée depuis laquelle s'étend le corps.

**13.** Système (1) selon l'une quelconque des revendications 11 et 12, dans lequel le boîtier (21) et le corps (40) sont solidarisés de manière amovible, le corps (40) étant réalisé en un matériau biocompatible, implantable dans la structure osseuse (2), la surface extérieure du corps (40) étant adaptée pour permettre l'ancrage du corps (40) dans la structure osseuse (2), le boîtier (21) comportant des organes de connexion électrique du générateur électrique (23) et du dispositif de mesure (24) aux première (11) et deuxième (12) électrodes.

**14.** Système (1) selon la revendication 11, dans lequel le boîtier et le corps sont réalisés en un matériau biocompatible, implantable à l'intérieur du sujet.

**Patentansprüche**

**1.** System (1) zur Bestimmung der Qualität einer Knochenstruktur (2) eines Individuums, insbesondere der Porosität einer Knochenstruktur (2), wobei das System (1) aufweist:

- einen Körper (10; 40), der geeignet ist, ein Loch in die Knochenstruktur (2) zu bohren, und eine Außenfläche (14) aufweist,
- mindestens eine erste Elektrode (11), die eine an der Außenfläche (14) des Körpers (10; 14) angeordnete Kontaktfläche hat, um bei der Bohrung in Kontakt mit der Knochenstruktur (2) zu kommen,
- mindestens eine zweite Elektrode (12), die eine an der Außenfläche (14) des Körpers (10; 14) angeordnete Kontaktfläche hat, um bei der Bohrung in Kontakt mit der Knochenstruktur (2) im Abstand von der ersten Elektrode (11) zu kommen,
- einen elektrischen Generator (23), der eingerichtet ist, während einer festgelegten Dauer einen elektrischen Strom zwischen der ersten (11) und zweiten (12) Elektrode anzulegen, wobei der elektrische Strom eine aus einer Spannung und einer Intensität ausgewählte Eigenschaft hat, die bekannt ist,
- eine Messungsvorrichtung (24), die eingerichtet ist, während der festgelegten Dauer die andere aus der Spannung und der Intensität ausgewählte Eigenschaft des elektrischen Stroms, der zwischen den Kontaktflächen der ersten (11) und der zweiten (12) Elektrode die Knochenstruktur (2) überquert, kontinuierlich zu messen,
- eine Verarbeitungsvorrichtung (28), die eingerichtet ist, während der festgelegten Dauer eine elektrische Größe, die die Fähigkeit der Knochenstruktur (2), den elektrischen Strom zwischen den Kontaktflächen der ersten (11) und zweiten (12) Elektrode fließen zu lassen, repräsentiert, ausgehend von der bekannten Eigenschaft und der gemessenen Eigenschaft kontinuierlich zu bestimmen, und während der festgelegten Dauer ein Signal, das die Qualität der Knochenstruktur (2), insbesondere die Porosität der Knochenstruktur (2) zwischen den Kontaktflächen der ersten (11) und zweiten (12) Elektrode repräsentiert, ausgehend von der bestimmten elektrischen Größe kontinuierlich bereitzustellen, **dadurch gekennzeichnet, dass** die Verarbeitungsvorrichtung (28) eingerichtet ist, während der festgelegten Dauer einen Wert für die elektrische Größe kontinuierlich zu berechnen und während der festgelegten Dauer den für die elektrische Größe berechneten Wert mit einem Wert für die Qualität der Knochenstruktur (2), insbesondere die Porosität der Knochenstruktur (2), kontinuierlich zu verknüpfen.

2. System (1) nach Anspruch 1, wobei der elektrische Generator (23) eingerichtet ist, den elektrischen Strom in Form mindestens eines elektrischen Pulses anzulegen.

3. System (1) nach Anspruch 1 oder 2, wobei die Verarbeitungsvorrichtung (28) eingerichtet ist, eine Transferfunktion zu speichern, die jeden der Werte eines Wertebereichs für die elektrische Größe mit einem Wert eines Wertebereichs für die Qualität der der Knochenstruktur (2), insbesondere die Porosität der Knochenstruktur (2), verknüpft.

4. System (1) nach einem der Ansprüche 1 bis 3, wobei die Verarbeitungsvorrichtung (28) eingerichtet ist, eine erste Beziehung zwischen der bekannten Eigenschaft und der gemessenen Eigenschaft aufzustellen und ausgehend von der ersten Beziehung die elektrische Größe zu bestimmen.

5. System (1) nach Anspruch 4, wobei die elektrische Größe aus der Leitfähigkeit und dem Widerstand der Knochenstruktur (2) zwischen den Kontaktflächen der ersten (11) und zweiten (12) Elektrode ausgewählt ist, wobei die Verarbeitungsvorrichtung (28) eingerichtet ist, um:

   - eine Distanz zwischen den Kontaktflächen der ersten (11) und zweiten (12) Elektrode zu bestimmen,
   - Abmessungen der Kontaktflächen des Kontakts der ersten (11) und zweiten (12) Elektrode mit der Knochenstruktur (2) zu bestimmen,
   - eine zweite Beziehung zwischen der Distanz und den Abmessungen aufzustellen, und
   - ausgehend von der ersten und zweiten Beziehung die elektrische Größe zu berechnen.

6. System (1) nach Anspruch 5, wobei die Verarbeitungsvorrichtung (28) eingerichtet ist, die Distanz zwischen den Kontaktflächen der ersten (11) und zweiten (12) Elektrode und die Abmessungen der Kontaktflächen des Kontakts der ersten (11) und zweiten (12) Elektrode mit der Knochenstruktur (2) zu speichern.

7. System (1) nach einem der Ansprüche 1 bis 6, wobei die Kontaktfläche mindestens einer der ersten (11) und zweiten (12) Elektrode eine Abmessung von mehr als 400$\mu$m aufweist.

8. System (1) nach einem der Ansprüche 1 bis 7, wobei die Verarbeitungsvorrichtung (28) eingerichtet ist, eine Gesamtheit von Werten für die elektrische Größe über die festgelegte Dauer zu speichern.

9. System (1) nach einem der Ansprüche 1 bis 8, wobei der Körper (10; 40) sich entlang einer zentralen Achse (A) bis zu einem distalen Ende (13) erstreckt, wobei sich mindestens eine der ersten (11) und zweiten (12) Elektrode sich parallel zu der zentralen Achse (A) erstreckt, wobei die Kontaktfläche an dem distalen Ende (13) dieser Elektrode an der Außenfläche (14) des Körpers (10; 40) erscheint.

10. System (1) nach einem der Ansprüche 1 bis 9, wobei mindestens eine der ersten (11) und zweiten (12) Elektrode sich im Innern des Körpers (10; 40) bis zu einem freien Ende, das die Kontaktfläche aufweist, erstreckt, wobei der Körper (10; 40) eine Schicht aus einem isolierenden Material (15) aufweist, die die Elektrode umgibt, derart, dass nur die Kontaktfläche an der Außenfläche (24) des Körpers (10; 40) erscheint.

11. System (1) nach einem der Ansprüche 1 bis 10, aufweisend ein mit dem Körper (10; 40) verbundenes Gehäuse (21) und eine Versorgungsvorrichtung (25), die zumindest den elektrischen Generator (23) und die Messungsvorrichtung (24) mit elektrischer Energie versorgt, wobei das Gehäuse (21) eingerichtet ist, zumindest den elektrischen Generator (23), die Messungsvorrichtung (24) und die Versorgungsvorrichtung (25) unterzubringen.

12. System (1) nach Anspruch 11, wobei das Gehäuse (21) einen Griff bildet, von dem aus sich der Körper erstreckt.

13. System (1) nach einem der Ansprüche 11 und 12, wobei das Gehäuse (21) und der Körper (40) auf lösbare Weise miteinander verbunden sind, wobei der Körper (40) aus einem biokompatiblen, in die Knochenstruktur (2) implantierbaren Material hergestellt ist, wobei die Außenfläche des Körpers (40) eingerichtet ist, um die Verankerung des Körpers (40) in der Knochenstruktur (2) zu erlauben, wobei das Gehäuse (21) Elemente zur elektrischen Verbindung des elektrischen Generators (23) und der Messungsvorrichtung (24) mit der ersten (11) und zweiten (12) Elektrode aufweist.

14. System (1) nach Anspruch 11, wobei das Gehäuse und der Körper aus einem biokompatiblen, im Innern des Individuums implantierbaren Material hergestellt sind.

## Claims

1. System (1) for determining the quality of a bone structure (2) of a subject, particularly the porosity of a bone structure (2), said system (1) comprising:

   - a body (10; 40) adapted for drilling into the bone structure (2) and presenting an outside surface (14),
   - at least one first electrode (11) having a contact surface arranged on the outside surface (14) of the body (10; 40) so as to come into contact with the bone structure (2) during drilling,
   - at least one second electrode (12) having a contact surface arranged on the outside surface (14) of the body (10; 40) so as to come into contact with the bone structure (2) at a distance from the first electrode (11) during drilling,
   - an electric generator (23) adapted for applying an electric current between the contact surfaces of the first (11) and second (12) electrodes for a determined period of time, said electric current having a characteristic chosen from among a voltage and an intensity which is known,
   - a measuring device (24) adapted for continuously measuring over the determined period of time the other characteristic chosen from among the voltage and the intensity of the electric current passing through the bone structure (2) between the contact surfaces of the first (11) and second (12) electrodes,
   - a processing device (28) adapted for continuously determining over the determined period of time an electrical magnitude representative of the capacity of the bone structure (2) for allowing the passage of the electric current between the contact surfaces of the first (11) and second (12) electrodes, based on the known characteristic and on the measured characteristic, and for continuously delivering over the determined period of time a signal representative of the quality of the bone structure (2), particularly of the porosity of the bone structure (2), between the contact surfaces of the first (11) and second (12) electrodes, based on the determined electrical magnitude,

   **characterized in that** the processing device (28) is adapted for continuously calculating a value for the electrical magnitude during the determined period of time, and for continuously associating during the determined period of time the value calculated for the electrical magnitude with a value for the quality of the bone structure, particularly for the porosity of the bone structure (2).

2. System (1) according to claim 1, wherein the electric generator (23) is adapted for applying the electric current in the form of at least one electric pulse.

3. System (1) according to claim 1 or 2, wherein the processing device (28) is adapted for storing a transfer function relating each of the values in a range of values for the electrical magnitude to a value in a range of values for the quality of the bone structure (2), particularly for the porosity of the bone structure (2).

4. System (1) according to any of claims 1 to 3, wherein the processing device (28) is adapted for establishing a first ratio between the known characteristic and the measured characteristic, and for determining the electrical magnitude based on said first ratio.

5. System (1) according to claim 4, wherein the electrical magnitude is chosen from among the conductivity and the resistivity of the bone structure (2) between the contact surfaces of the first (11) and second (12) electrodes, the processing device (28) being adapted to:

   - determine a distance between the contact surfaces of the first (11) and second (12) electrodes,
   - determine dimensions of the contact surfaces of the first (11) and second (12) electrodes with the bone structure (2),
   - establish a second ratio between said distance and said dimensions, and
   - calculate the electrical magnitude based on the first and second ratios.

6. System (1) according to claim 5, wherein the processing device (28) is adapted for storing the distance between the contact surfaces of the first (11) and second (12) electrodes, and the dimensions of the contact surfaces of the first (11) and second (12) electrodes with the bone structure (2).

7. System (1) according to any of claims 1 to 6, wherein the contact surface of at least one of the first (11) and second (12) electrodes has a dimension greater than 400 $\mu$m.

8. System (1) according to any of claims 1 to 7, wherein the processing device (28) is adapted for storing a set of values for the electrical magnitude over the determined period of time.

9. System (1) according to any of claims 1 to 8, wherein the body (10; 40) extends along a central axis (A) to a distal end (13), with at least one of the first (11) and second (12) electrodes extending parallel to the central axis (A), the contact surface of said electrode being flush with the outside surface (14) of the body (10; 40) at the distal end (13).

**10.** System (1) according to any of claims 1 to 9, wherein at least one of the first (11) and second (12) electrodes extends inside the body (10; 40) to a free end presenting the contact surface, said body (10; 40) comprising a layer of insulating material (15) surrounding said electrode such that only said contact surface is level with the outside surface (14) of the body (10; 40).

**11.** System (1) according to any of claims 1 to 10, comprising a casing (21) attached to the body (10; 40) and a power supply device (25) supplying electrical energy to at least the electric generator (23) and the measuring device (24), said casing (21) being adapted to receive at least the electric generator (23), the measuring device (24) and the power supply device (25).

**12.** System (1) according to claim 11, wherein the casing (21) forms a handle from which the body extends.

**13.** System (1) according to any of claims 11 and 12, wherein the casing (21) and the body (40) are attached together in a detachable manner, the body (40) being made of a biocompatible material which can be implanted in the bone structure (2), the outside surface of the body (40) being adapted to allow anchoring the body (40) in the bone structure (2), the casing (21) comprising means for electrically connecting the electric generator (23) and the measuring device (24) to the first (11) and second (12) electrodes.

**14.** System (2) according to claim 11, wherein the casing and the body are made of a biocompatible material, implantable inside the subject.

FIG. 1

FIG. 2

**FIG. 3**

**FIG. 4**

FIG. 5

FIG. 6a

FIG. 6b

**FIG. 7**

FIG. 8a

FIG. 8b

EP 2 640 265 B1

FIG. 9

FIG. 10

EP 2 640 265 B1

## RÉFÉRENCES CITÉES DANS LA DESCRIPTION

**Documents brevets cités dans la description**

- WO 2008119992 A
- US 6997883 B
- WO 2009152244 A
- DE 102004035001
- WO 03068076 A